# FASCICULE DE BREVET EUROPEEN

(11) **EP 2 874 596 B1**
(45) Date de publication et mention de la délivrance du brevet: **06.03.2019**
(21) Numéro de dépôt: 13733363.9
(22) Date de dépôt: 06.06.2013
(51) Int. Cl.: A61K 8/9789, A61K 8/34, A61Q 5/00, A61Q 17/00, A61Q 19/00, A61K 8/60

(54) **EXTRAIT DE FIBRES DE COTON ET COMPOSITION COSMÉTIQUE ET LEUR UTILISATION POUR PROTÉGER, NOURRIR ET HYDRATER LA PEAU**
BAUMWOLLFASEREXTRAKT UND KOSMETISCHE ZUBEREITUNG UND DAVON VERWENDUNG ZUM SCHUTZ, ZUR NÄHRUNG UND FEUCHTIGKEITSVERSORGUNG DER HAUT
EXTRACT OF COTTON FIBRES AND COSMETIC COMPOSITION AND USE THEREOF FOR PROTECTING, NOURISHING AND HYDRATING THE SKIN

(30) Priorité: 11.06.2012 FR 1201664
(43) Date de publication de la demande: 27.05.2015
(73) Titulaire: ISP Investments LLC, Wilmington, DE 19805 (US)
(72) Inventeur: DOMLOGE, Nouha, F-06650 OPIO (FR); PORTOLAN, Frédérique, F-06560 Valbonne (FR); BOTTO, Jean-Marie, 06560 Valbonne (FR)
(74) Mandataire: Miquel, Corinne
(86) Numéro de dépôt international: PCT/FR2013/051298
(87) Numéro de publication internationale: WO 2013/186465

(56) Documents cités:
- EP-A1- 1 930 012
- EP-A2- 1 179 339
- WO-A1-2011/026039
- WO-A2-02/38110
- DE-U1-202008 014 068
- FR-A1- 2 847 815
- FR-A1- 2 947 727
- JP-A- 2008 001 599

## Description

L'invention concerne le domaine de la cosmétique.

La présente invention a pour objet un extrait de fibres de coton comprenant un hydrolysat de fibres de coton constitué de mono, di, tri et tétra saccharides, et une composition cosmétique comprenant comme principe actif, dans un milieu physiologiquement adapté, au moins un extrait de fibres de coton.

La peau est le substrat kératinique majeur de l'organisme. C'est un organe vital recouvrant la totalité de la surface du corps et assurant des fonctions multiples telles que des fonctions sensitives, protectrices vis-à-vis d'agressions externes multiples, immunitaires, métaboliques ou encore thermorégulatrices. Ces rôles sont rendus possibles grâce à une structure complexe qui associe des structures tissulaires variées.

La peau est constituée de trois compartiments distincts superposés : l'épiderme, le derme et l'hypoderme. L'épiderme est un épithélium de revêtement qui constitue la structure externe de la peau et assure sa fonction de protection. Cette fonction est assurée par la cohésion des cellules épithéliales et par la production d'une protéine filamenteuse et résistante : la kératine. L'épiderme est caractérisé par une organisation en strates correspondant à un état de différenciation croissant des kératinocytes, depuis la zone la plus profonde (stratum basale) vers la zone la plus superficielle (stratum corneum). Pendant leurs migrations vers la surface, les kératinocytes s'aplatissent et la synthèse de kératines, une famille de protéines fibreuses, s'intensifie. En progressant vers les couches supérieures, les kératinocytes s'aplatissent encore, leur noyau commence à dégénérer. Le contenu cellulaire riche en lipides, cholestérol, acides gras libres saturés et céramides, est alors excrété dans l'espace extracellulaire, ce qui augmente la cohésion entre les cellules et contribue au rôle de barrière de l'épiderme.

La couche cornée (stratum corneum) est ainsi très résistante aux agressions externes. La kératine est la composante essentielle de tous les substrats kératiniques et en particulier les fibres capillaires, les poils, les ongles et autres phanères.
Le cheveu, ou le poil, est une structure capillaire constituée de cellules mortes remplies de filaments de kératine et de résidus lipidiques provenant des membranes plasmiques. Chaque cheveu est produit par un follicule pileux, lui-même constitué d'un bulbe, d'une tige pilaire et d'une glande sébacée. Dans le bulbe, ce sont les kératinocytes situés à la périphérie qui constituent l'assise proliférative. Lors de la différenciation, les kératinocytes se déplacent vers le centre du follicule pour constituer la tige pilaire et se chargent en fibres de kératine, ce qui rend le cheveu très résistant.

Les professionnels de la santé et de la cosmétique recherchent depuis de nombreuses années des moyens d'entretenir les substrats kératiniques, notamment la peau et les cheveux, ainsi que des moyens d'augmenter la résistance de la peau et des cheveux aux agressions extérieures et au stress qu'ils subissent quotidiennement.

Un certain nombre de substances introduites dans des produits cosmétiques ou pharmaceutiques ont vu le jour, mais il existe toujours un besoin, de développer de nouveaux ingrédients permettant de protéger les substrats kératiniques, de renforcer la barrière cutanée et de limiter l'impact des agressions extérieures. Par ailleurs, les inventeurs se sont donné comme objectif de mettre au point un produit cosmétique d'origine végétale, de façon à répondre aux attentes des consommateurs et aux exigences croissantes d'innocuité.

Le problème technique à résoudre a donc été, pour les inventeurs, de trouver un nouveau produit naturel d'origine végétale, physiologiquement acceptable, qui soit capable d'apporter de véritables soins aux substrats kératiniques mais aussi de protéger la peau et les cheveux d'une manière efficace afin qu'ils ne subissent pas les dégradations occasionnées par les agressions et les stress d'origine extérieure.

Les inventeurs ont réussi à sélectionner un produit naturel d'origine végétale qui, traité d'une manière appropriée, présente des propriétés remarquables lorsqu'il est appliqué sur un substrat kératinique. De manière inattendue, les inventeurs ont découvert qu'un extrait de fibres de coton comprenant un hydrolysat de fibres de coton constitué exclusivement de mono, di, tri et tétra saccharides présente des propriétés remarquables au niveau des substrats kératiniques et, notamment, qu'il protège la peau et les cheveux.

Le coton est l'ensemble des fibres (ou poils) recouvrant la graine du cotonnier. Le cotonnier, ou Gossypium, est une dicotylédone de la famille des malvacées. On compte de 40 à 50 espèces vivaces ou annuelles, ligneuses ou herbacées. Seules quatre d'entre elles sont cultivées pour leur fibre : *Gossypium hirsutum, Gossypium barbadense, Gossypium herbaceum* et *Gossypium arboreum.* Ces quatre espèces de cotonniers ont donné naissance à de nombreuses variétés hybrides, classées selon la longueur de leur fibre, la pubescence de la graine et la forme des bractées. C'est la lignée des cotonniers *Gossypium hirsutum,* qui fournit à lui seul de 80 à 90 % de la production mondiale de coton. Le fruit du cotonnier est une capsule ovale, coriace, et renfermant de très nombreuses graines portant des poils (ou fibres) serrés de longueurs variées. C'est à partir de ces fibres, traitées de manière appropriée, que les inventeurs ont mis au point le nouvel extrait de fibres de coton et la composition cosmétique selon l'invention.

A la connaissance de la demanderesse, il n'a jamais été décrit dans l'art antérieur l'utilisation d'un produit à base de fibres de coton hydrolysées dans des compositions cosmétiques. Jusqu'à présent seules les huiles des graines de coton ainsi que des fibres de coton ont été utilisées en cosmétique, comme par exemple dans le brevet US 5,466,441 ou dans le brevet FR2799647.

Le brevet FR2799647 divulgue des compositions sous forme d'émulsion contenant des fibres, au moins un tensioactif siliconé et au moins une cire, pour traiter, protéger, soigner, démaquiller et/ou nettoyer la peau, les lèvres et/ou les cheveux. Les fibres de coton sont citées sans être exemplifiées, ni préférées. Les fibres préférées sont synthétiques et présentent une longueur comprise entre 0,1 et 1,5 mm.

Il est également connu des compositions à base de miellat de coton dans la demande de brevet WO2004052331 pour les soins des substrats kératiniques, ledit miellat de coton étant une substance sécrétée par des insectes et présente sur le coton. Il contient essentiellement des sucres synthétisés par les insectes et des sucres d'origine physiologique, notamment du glucose, du fructose, du saccharose, du tréhalose, du tréhalulose, du mélézitoze et de l'inositol EP 1930012 divulgue des composition comprenant un cellooligosaccharid.

Ainsi, selon un premier aspect, la présente invention a pour objet un extrait de fibres de coton comprenant un hydrolysat de fibres de coton constitué exclusivement de monosaccharides et/ou disaccharides et/ou trisaccharides et/ou tétrasaccharides.

Par « hydrolysat de fibres de coton » selon la présente invention, on entend le produit résultant de l'hydrolyse de fibres de coton via une action enzymatique, notamment une cellulase.

Par « extrait de fibres de coton » selon la présente invention, on entend une solution aqueuse comprenant un hydrolysat de fibres de coton hydrolysé d'une manière enzymatique produite selon le procédé décrit ci-après.

Par « saccharides » selon la présente invention, on entend tous glucides qui sont une classe de molécules organiques contenant un groupement carbonyle (aldéhyde ou cétone) et plusieurs groupements hydroxyle (-OH). On entend particulièrement tous glucides comprenant six atomes de carbone.

Par «monosaccharides et/ou disaccharides et/ou trisaccharides et/ou tétrasaccharides» selon la présente invention, on entend tous saccharides comprenant un saccharide ou bien deux, trois ou quatre saccharides liés entre eux.

L'extrait de fibres de coton comprend un hydrolysat de fibres de coton hydrolysé d'une manière enzymatique selon le procédé décrit ci-après.

Dans un mode de réalisation préféré de l'extrait de fibres de coton selon l'invention, les monosaccharides, disaccharides, trisaccharides et/ou tétrasaccharides sont le glucose, le cellobiose, le cellotriose, le cellotétraose et les oligomères de cellulose.

Dans un mode de réalisation particulièrement préféré selon l'invention, l'extrait de fibres de coton comprend en outre au moins un saccharide supplémentaire. Le ou les saccharides sont de préférence d'origine naturelle, et de préférence le glucose et/ou le fructose et/ou le tréhalose et/ou le saccharose et/ou l'inositol.

L'extrait de fibres de coton comprend avantageusement en outre les saccharides supplémentaires suivants : le glucose, le fructose, le tréhalose le saccharose et l'inositol.

Encore plus avantageusement, l'extrait de fibres de coton de l'invention comprend environ 2 % d'hydrolysat de fibres de coton, 0,8 % de glucose, 0,8 % de fructose, 1,1 % de tréhalose, 1,7 % de saccharose et 0,5 % d'inositol.

Selon un second aspect, la présente invention a pour objet une composition cosmétique comprenant comme principe actif, dans un milieu physiologiquement adapté, un extrait de fibres de coton tel que décrit ci-après.

Par « un milieu physiologiquement adapté », on entend un milieu compatible avec la peau, les poils ou les cheveux.

La composition cosmétique selon l'invention comprend en tant que principe actif un produit naturel d'origine végétal, l'extrait de fibres de coton, lequel a été hydrolysé d'une manière enzymatique selon le procédé décrit ci-après. Les inventeurs ont démontré, d'une manière surprenante, que les compositions cosmétiques comprenant en tant que principe actif un extrait de fibres de coton selon l'invention, permettent notamment de protéger les cellules contre les chocs osmotiques, d'obtenir une protection contre les dommages causés à l'ADN des cellules, notamment que les compositions cosmétiques permettent de protéger l'ADN des cellules lorsque celles-ci sont soumises à des stress tels que, par exemple, la privation de nutriments. Ainsi, les inventeurs ont démontré que les compositions cosmétiques présentent un effet protecteur sur les kératinocytes et sur l'ADN et permettent notamment d'hydrater la peau.

Ces propriétés protectrices des compositions cosmétiques selon l'invention peuvent être utilisées, par exemple, pour protéger la peau et/ou les cheveux contre les agressions extérieures provoquées, notamment, par l'action du rayonnement solaire ou par d'autres agents physiques, chimiques ou biologiques. Ces propriétés protectrices peuvent aussi être utilisées pour lutter contre le vieillissement de la peau.

Il est bien entendu que le principe actif selon l'invention peut être utilisé seul ou bien en association avec d'autres principes actifs.

Selon l'invention, on peut ajouter à la composition cosmétique selon l'invention d'autres principes actifs destinés notamment à la prévention et/ou au traitement des manifestations cutanées du vieillissement et/ou à la protection de la peau et/ou des cheveux contre les agressions extérieures.

Ainsi, les compositions cosmétiques utilisables selon l'invention peuvent contenir, en outre, au moins un autre principe actif destiné à renforcer l'action de l'extrait de fibres de coton selon l'invention, dans le domaine de la prévention et de l'amélioration des signes cutanés du vieillissement ou permettant d'élargir la gamme de propriétés de la composition cosmétique selon l'invention.

On peut citer, de manière non limitative, les classes d'ingrédients suivantes dans le domaine cosmétique: les agents régénérants, anti-âges, antirides, apaisants, antioxydants, cicatrisants, relipidants, nutritifs, anti-radicalaires, anti-glycation, hydratants, antibactériens, antifongiques, kératolytiques, myorelaxants, desquamants, raffermissants, les agents stimulant la synthèse de macromolécules dermiques ou le métabolisme énergétique ou la microcirculation ou la pousse des ongles ou la pousse des cheveux, les agents modulant la différenciation ou la pigmentation de l'épiderme, les agents inhibant les métallo-protéinases ou les filtres solaires.

Dans un mode plus particulier de réalisation, la composition cosmétique selon l'invention comprendra, outre l'extrait de fibres de coton selon l'invention,
- au moins un composé activateur du cytochrome c et/ou,
- au moins un composé hydratant, tel qu'un composé activateur des aquaporines et/ou,
- au moins un composé activateur des sirtuines et/ou,
- au moins un composé qui augmente l'adhésion cellulaire et/ou,
- au moins un composé qui augmente la production de protéines matricielles telles que le collagène, fibronectine, laminine, glycosaminoglycanes et/ou,
- au moins un composé modulateur de l'activité du protéasome et/ou,
- au moins un composé modulateur du rythme circadien et/ou,
- au moins un composé modulateur des protéines HSP et/ou,
- au moins un composé qui augmente l'énergie cellulaire et/ou,
- au moins un composé modulant la pigmentation de la peau et/ou,
- au moins un composé activateur du coenzyme Q10 et/ou,
- au moins un composé améliorant la fonction barrière, tel qu'un composé activateur de la transglutaminase ou de la HMG-CoA réductase et/ou,
- au moins un composé protecteur de la mitochondrie.

Lesdits composés ci-dessus peuvent être d'origine naturelle, comme des extraits peptidiques de plantes, ou encore d'origine synthétique, comme des peptides.

Indépendamment de leurs fonctions, les autres agents actifs ou principes actifs associés à l'agent actif ou principe actif selon l'invention dans la composition pourront avoir des structures chimiques très diverses. On peut citer de manière non limitative, les peptides, la vitamine C et ses dérivés, les vitamines du groupe B, la DHEA (dihydroépiandrostérone), les phytostérols, l'acide salicylique et ses dérivés, les rétinoïdes, les flavonoïdes, les amines de sucres, les azoles, les sels métalliques, l'allantoïne, les extraits peptidiques d'origine végétale ou encore les polymères.

Par ailleurs, la composition cosmétique selon l'invention peut comprendre en outre, au moins un composé améliorant la santé des cheveux.

On peut citer notamment des vitamines, d'autres extraits peptidiques végétaux, le minoxidil, des esters de l'acide nicotinique, des oligo-éléments, des agents anti-inflammatoires, de l'acide rétinoïque ou ses dérivés, du rétinol, des inhibiteurs de la 5α-réductase ou des composés peptidiques issus de la synthèse chimique. Comme exemple de vitamine on peut citer les vitamines A, E, B5, B6, C, H, ou PP, comme exemple d'oligo-éléments on peut citer le zinc, le cuivre, le magnésium, ou encore le silicium.

Selon un mode de réalisation avantageux de l'invention, l'extrait de fibres de coton est présent dans la composition cosmétique à une concentration comprise entre 0,01 % et 20 % en poids par rapport au poids total de la composition, et préférentiellement à une concentration comprise entre 0,1 % et 10 % en poids par rapport au poids total de la composition.

Selon un mode de réalisation particulièrement avantageux de l'invention, la composition cosmétique selon l'invention comprend également une quantité comprise entre 0,01 % et 5 % en poids par rapport au poids total de la composition d'au moins un saccharide supplémentaire tel que le glucose et/ou le fructose et/ou le tréhalose et/ou le saccharose et/ou l'inositol.

Par « au moins un saccharide supplémentaire » selon la présente invention, on entend que le principe actif, en plus des monosaccharides et/ou disaccharides et/ou trisaccharides et/ou tétrasaccharides, comprend des saccharides que l'on ajoute à la composition initialement obtenue par hydrolyse enzymatique des fibres de coton. Ces saccharides sont préférentiellement le glucose et/ou le fructose et/ou le tréhalose et/ou le saccharose et/ou l'inositol.

Dans un mode de réalisation encore plus avantageux de l'invention, la composition cosmétique selon l'invention comprend environ 2 % d'extrait de fibres de coton, 0,8 % de glucose, 0,8 % de fructose, 1,1 % de tréhalose, 1,7 % de saccharose et 0,5 % d'inositol.

Les compositions cosmétiques selon la présente invention sont destinées aux soins des substrats kératiniques.

Dans l'invention, on entend, par l'expression «substrat kératinique», tous les substrats qui sont composés en grande partie de kératine. Ce sont des substrats tels que la peau, les cheveux, les cils, les sourcils ou encore les ongles et les phanères en général. Par les soins des substrats kératiniques, on entend toutes les actions destinées à conserver ou à rétablir un bon fonctionnement de ce substrat, ou encore tout moyen qui sert à préserver ou à améliorer son apparence. Ainsi le soin inclus l'hydratation, l'apaisement, la protection contre tous types d'agressions, notamment la protection solaire, la lutte et la prévention des manifestations du vieillissement, notamment les manifestations cutanées du vieillissement.

Par « manifestations cutanées du vieillissement » on entend toutes modifications de l'aspect extérieur de la peau dues au vieillissement comme, par exemple, les rides et ridules, la peau flétrie, la peau molle, la peau amincie, le manque d'élasticité et/ou de tonus de la peau, la peau terne et sans éclat mais également toutes modifications internes de la peau qui ne se traduisent pas systématiquement par un aspect extérieur modifié comme, par exemple, toutes dégradations internes de la peau consécutive à une exposition aux rayonnements ultra-violets.

Pour le nettoyage et/ou le lavage des cheveux et/ou de la peau, l'utilisation de lotions détergentes (tels que des shampooings et autre savons) à base essentiellement d'agents tensioactifs classiques notamment de type anionique, non-anionique et/ou amphotère, mais plus particulièrement de type anionique, est courante. Ces compositions sont appliquées sur les substrats kératiniques mouillés et la mousse générée par le massage permet, après rinçage à l'eau, l'élimination des différentes salissures initialement présentes. Ces compositions possèdent, certes, un bon pouvoir lavant mais les propriétés cosmétiques qui leurs sont attachées restent toutefois assez faibles compte tenu du caractère relativement agressif d'un tel traitement nettoyant. En effet, ce traitement peut, à la longue, occasionner sur la fibre capillaire et/ou sur la peau des dommages plus ou moins marqués, liés en particulier à une élimination progressive des protéines contenues dans ou à la surface de ces dernières. Ainsi, pour améliorer les propriétés cosmétiques des compositions détergentes ci-dessus, une solution est d'introduire des agents cosmétiques complémentaires destinés principalement à réparer ou à limiter les effets néfastes ou indésirables induits par les différents traitements ou agressions que subissent les fibres capillaires et la peau, c'est à dire à protéger. Ces agents cosmétiques pourront être, par exemple, un extrait de fibres de coton selon l'invention ou une composition cosmétique selon l'invention.

Les compositions cosmétiques selon l'invention sont destinées à améliorer l'aspect et la qualité des substrats kératiniques de l'individu qui en fait usage.

Les compositions cosmétiques selon la présente invention se présentent sous une forme galénique adaptée à l'administration par voie topique cutanée et adaptée à une administration sur les cheveux. Elle couvre toutes les formes cosmétiques. Ces compositions doivent donc contenir un milieu physiologiquement acceptable, c'est-à-dire compatible avec la peau, les poils ou les cheveux. Ces compositions peuvent notamment se présenter sous forme d'une solution aqueuse, hydro-alcoolique ou huileuse ; d'une émulsion huile-dans-eau, eau-dans-huile ou émulsions multiples ; elles peuvent aussi se présenter sous forme de crèmes, de suspensions ou encore de poudres, adaptées à une application sur la peau, les muqueuses, les lèvres et/ou les cheveux. Ces compositions peuvent être plus ou moins fluides et avoir l'aspect d'une crème, d'une lotion, d'un lait, d'un sérum, d'une pommade, d'un shampooing, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Ces compositions comprennent, en outre, tout additif usuellement utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques, des huiles essentielles, des vitamines, des acides gras essentiels, des tensioactifs, des polymères filmogènes, etc....Dans tous les cas, l'homme du métier veillera à ce que ces adjuvants ainsi que leurs proportions soient choisis de telle manière à ne pas nuire aux propriétés avantageuses recherchées de la composition cosmétique selon l'invention. Ces adjuvants peuvent, par exemple, être présents à une concentration de 0,01 à 20 % du poids total de la composition.

Lorsque la composition cosmétique de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition. Bien entendu, l'homme du métier veillera à choisir les éventuels composés complémentaires, actifs ou non-actifs, et/ou leurs quantités, de telle sorte que les propriétés avantageuses du mélange ne soient pas ou sensiblement pas, altérées par l'adjonction envisagée.

Les compositions selon l'invention trouvent une application notamment comme composition cosmétique pour la peau, les muqueuses et/ou les semi-muqueuses, mais aussi comme composition cosmétique pour les cheveux et/ou les poils. On peut également envisager une application dans le domaine des compositions de maquillage de la peau du visage et du corps, telles que les rouges à lèvres, les fonds de teint, les crèmes teintées, les sticks anti-cernes, les compositions anti-solaires ou de bronzage artificiel.

Les compositions, objet de l'invention, trouvent leurs applications dans grand nombre d'applications cosmétiques, notamment pour le traitement, la protection, le soin, le démaquillage et/ou le nettoyage de la peau, des lèvres et/ou des cheveux, et/ou pour le maquillage de la peau, des lèvres, des cils et/ou du corps.

Les compositions cosmétiques selon l'invention peuvent également consister en des préparations solides comprenant également des savons ou des pains de nettoyage.

Les compositions cosmétiques peuvent être aussi conditionnées sous forme d'une composition pour aérosol comprenant également un agent propulseur sous pression. Les compositions peuvent être aussi à usage bucco-dentaire, par exemple une pâte de dentifrice.

La composition cosmétique selon l'invention peut être destinée à former un filtre protecteur au niveau des substrats kératiniques.

L'invention a pour troisième objet une composition pour une utilisation dans une méthode pour protéger les substrats kératiniques, et plus particulièrement pour protéger la peau et les cheveux, contre tous les types d'agressions extérieures consistant à appliquer sur la surface des substrats kératiniques, et plus particulièrement sur la peau et/ou les cheveux, une quantité efficace d'une composition cosmétique selon l'invention.

L'invention concerne également un procédé de traitement cosmétique pour renforcer la barrière cutanée de la peau et/ou pour renforcer la protection des cheveux et/ou pour augmenter la synthèse de kératine et/ou pour nourrir les substrats kératiniques, et plus particulièrement la peau et les cheveux, consistant à appliquer sur la surface de la peau et/ou des cheveux, une quantité efficace d'une composition cosmétique selon l'invention.

L'invention a pour quatrième objet un procédé de préparation d'un extrait de fibres de coton comprenant les étapes suivantes :
a- des fibres de coton réduites en poudre sont agitées dans de l'eau osmosée en présence de cellulases,
b- l'hydrolyse s'effectue pendant une durée comprise entre 15 et 24 heures à une température d'environ 50°C, pour obtenir un hydrolysat des fibres de coton
c- les cellulases sont désactivées par chauffage à 80°C pendant environ deux heures,
d- l'hydrolysat obtenu est filtré éventuellement concentré ou dilué dans de l'eau osmosée et du glycérol.

Selon un mode de réalisation particulier de l'invention, l'étape b de l'hydrolyse s'effectue pendant une durée d'environ 18 heures.

Selon le procédé de préparation d'un extrait de fibres de coton selon l'invention, il est procédé aux étapes décrites ci-dessous :
- Les fibres de coton (genre Gossypium, préférentiellement *Gossypium hirsutum, Gossypium herbaceum,* et encore plus préférentiellement *Gossypium hirsutum*) sont réduites en poudre. Alternativement on peut utiliser des fibres brutes prétraitées par une solution de soude à 14 % pendant au moins 12 heures.
- Une proportion en poids de 10 à 20 % de fibres est mise à agiter en présence d'eau (10 % min - 20 % max), puis soumise à une hydrolyse enzymatique par des cellulases.
- Les enzymes sont ensuite désactivées par chauffage.
- Le mélange est filtré, concentré ou dilué pour obtenir une concentration comprise entre 20 et 50 g/kg de saccharides de type monosaccharides et/ou disaccharides et/ou trisaccharides et/ou tétrasaccharides et plus particulièrement du glucose et/ou cellobiose et/ou cellotrioses et/ou cellotétraose et/ou oligomères de cellulose.
- Environ 30 % en poids de glycérol est ajouté.
- Le mélange est filtré par filtration sur plaques de porosité décroissante jusqu'à 0.2 µm et pasteurisé à basse température.

Dans un mode particulier de réalisation selon l'invention, un ou plusieurs sucres purs (saccharides), d'origine végétale peuvent être ajoutés, avant l'étape de filtration dans les proportions indiquées ci-après:

| | |
|---|---|
| Glucose | 0,5 - 1,5 % |
| Fructose | 0,5 - 1,5 % |
| Trehalose | 1,0 - 1,5 % |
| Saccharose | 1,5 - 2,5 % |
| Inositol | 0,3 - 0,7 % |

La concentration finale en sucres totaux de l'extrait de fibres de coton, supplémenté ou non en sucres, est comprise entre 55 et 75 g/kg.

L'invention a pour cinquième objet l'utilisation d'un extrait de fibres de coton obtenu selon le procédé de préparation décrit ci-dessus pour la préparation d'une composition cosmétique selon l'invention.
La présente invention va maintenant être illustrée au moyen des exemples suivants, qui ne sauraient limiter la portée de la présente invention.

### Exemple 1 - Procédé de préparation de l'extrait de fibres de coton

14,3 kg de fibres de coton (espèce *Gossypium hirsutum*) réduites en poudre sont mis à agiter dans qsp 100 kg d'eau osmosée en présence de 1,15 kg d'enzyme de type cellulase, à savoir (Celluclast®CL).

L'hydrolyse est réalisée en maintenant ce mélange pendant 18h à 50°C. Puis les enzymes sont désactivées par chauffage à 80 °C pendant 2 heures et le mélange est filtré (Etape de filtration standard, préférentiellement filtre cloche).

A ce stade, l'extrait contient uniquement des sucres, à une concentration de 40 g/Kg de sucres totaux. Le dosage des sucres totaux est effectué par la technique Sucre-phénol.

Cet extrait est ensuit filtré et dilué pour aboutir à une concentration finale de 20 g/Kg de sucres totaux.

L'analyse par chromatographie sur couche mince, montre que l'extrait contient des monosaccharides, disaccharides, trisaccharides et tétrasaccharides et plus particulièrement du glucose, cellobiose, cellotrioses, cellotétraose et oligomères de cellulose.

L'extrait est dilué dans un mélange d'eau osmosée et 30 % de glycérol afin d'obtenir une concentration de sucres totaux de 20 g/kg.

Le mélange est filtré par filtration sur plaques de porosité décroissante jusqu'à 0.2 µm. et pasteurisé à basse température.

Les sucres suivants sont ajoutés

| | |
|---|---|
| Glucose | (0,8 %) |
| Fructose | (0,8 %) |
| Trehalose | (1,1 %) |
| Saccharose | (1,7 %) |
| Inositol | (0,5 %) |

La concentration finale de l'extrait est de 60 g/kg de sucres totaux.

### Exemple 2 - Mise en évidence de l'effet de l'extrait de fibres de coton sur la synthèse de kératine.

Les expériences ont été réalisées sur des coupes de peau humaine mises en culture pendant 24 heures. L'expression des kératines a été étudiée, sur ces coupes de peau, grâce à la méthode de marquage par immunofluorescence.

L'extrait de fibres de coton préparé à l'exemple 1 a été dilué dans un tampon « phosphate buffer salin » (PBS) pour obtenir une concentration finale de 0,5 % puis la dilution a été appliquée sur les coupes de peau, à raison de deux applications. L'effet de l'extrait a été évalué par comparaison avec une coupe de peau non traitée avec l'extrait de fibres de coton selon l'invention.

Après application, les échantillons de peau ont été cultivés pendant 24 heures, puis préparés pour l'inclusion dans la paraffine. L'immuno-marquage a ensuite été réalisé à l'aide d'un anticorps anti-kératine. La détection de la quantité de kératine a été réalisée par l'immunofluorescence. Ces études ont démontré une augmentation nette de la fluorescence de la kératine sur les coupes de peau traitées avec l'extrait de fibres de coton par rapport aux coupes non traitées. Ces résultats révèlent une augmentation importante de la synthèse de kératine.

### Exemple 3 - Mise en évidence de l'effet protecteur de l'extrait de fibres de coton sur des kératinocytes.

L'étude a été réalisée sur des kératinocytes humains HaCaT, en phase de croissance exponentielle dans des LabtecksTM. Les kératinocytes ont été ensemencés dans des plaques de 96 puits.

Des cellules sont prétraitées durant 2 heures en présence d'extrait à 0,5 %. La condition contrôle réalisée est constituée de cellules non traitées par l'extrait selon l'invention. Puis, le milieu de culture, contenant ou ne contenant pas l'extrait, est remplacé par du PBS pendant une période de 3h.

Des tests de viabilité cellulaire ont été réalisés grâce au test MTT. D'une manière générale, l'agent MTT est utilisé pour évaluer la cytotoxicité d'un produit vis-à-vis d'un milieu cellulaire par mesure de la viabilité cellulaire.

Les kératinocytes sont incubés dans une solution contenant 0,1 mg/ml de MTT (3-[4, 5-diméthylthiazol-2-yl]-2, 5-diphényltétrazolium bromide). Ce composé est absorbé par les cellules vivantes puis métabolisé par les enzymes mitochondriales (succinate déshydrogénase) en un composé bleu violet, le formazan, qui se présente sous forme de cristaux violets insolubles en milieu aqueux.

Les cristaux de formazan sont solubilisés dans du DMSO, ils donnent une densité optique (D.O.) proportionnelle au nombre de cellules vivantes présentes dans l'échantillon. Des mesures de densité optique ont ensuite été réalisées pour chaque échantillon étudié (La D.O. se lisant à 540 nm). La D.O. est alors directement proportionnelle à l'activité enzymatique ainsi qu'au nombre de cellules vivantes.

Les résultats ont démontré que les cellules cultivées dans du PBS, c'est-à-dire totalement privées de nutriments, subissent un stress très intense et s'altèrent rapidement.

De la même manière, ces mesures ont démontré que, selon les concentrations d'extrait utilisées, les cellules traitées avec ledit extrait ont une viabilité qui augmente de 15 % à 30 % par rapport aux cellules non traitées. Ces résultats démontrent ainsi clairement que l'extrait de fibres de coton selon l'invention possède un pouvoir protecteur important au niveau cellulaire.

### Exemple 4 - Mise en évidence de l'effet protecteur de l'extrait de fibres de coton contre le choc osmotique.

L'étude a été réalisée sur des kératinocytes humains HaCat, en phase de croissance exponentielle dans des LabtecksTM. Les cellules ont ensuite été traitées pendant 1 heure en présence de 0,5 % de l'extrait de fibres de coton. Puis le milieu a été retiré et les kératinocytes ont été incubés pendant 1 heure dans un milieu hypertonique (c'est-à-dire contenant du DMEM et 500 mM de NaCl).,

Puis un test MTT (tel que décrit ci-dessus) a été réalisé afin d'étudier la viabilité cellulaire.

Le résultat de ce test démontre que la viabilité des cellules est diminuée de moitié lorsque celles-ci sont soumises à un choc osmotique. En outre, les résultats démontrent que le prétraitement avec l'extrait de fibres de coton augmente de 32 % la viabilité de kératinocytes par rapport aux cellules témoins, c'est-à-dire non traitées avec l'extrait mais ayant subi le choc osmotique.

### Exemple 5 - Mise en évidence de l'effet de l'extrait de fibres de coton sur la protection de l'ADN.

Le même test que celui de l'exemple 3 a été effectué : les cellules ont été soumises à un stress provoqué par une privation de nutriments ("food starvation").

Suite à ce stress, un test des Comètes a été réalisé afin d'évaluer la dégradation de l'ADN des cellules traitées avec l'extrait de fibres de coton par comparaison avec les cellules non traitées avec l'extrait.

Le test des Comètes ou "Single Cell Gel Electrophoresis" est une technique d'électrophorèse sur microgel d'agarose rapide qui permet de visualiser les cassures de l'ADN simple et double-brin sur des cellules individuelles.

Après traitement, les cellules sont emprisonnées dans un gel d'agarose et lysées dans un tampon à forte salinité contenant des détergents. L'ADN est ensuite dénaturé par bain alcalin suivi d'une courte électrophorèse, puis il est mis en évidence par l'iodure de propidium. L'ADN d'une cellule altérée s'étire vers l'anode proportionnellement au nombre de cassures, en prenant plus ou moins la forme d'une comète. L'ADN fortement dégradé se retrouve dans la "queue" de la comète. Une cellule intacte reste ronde, l'ADN restant compacté au niveau de la "tête" de la comète. L'évaluation des lésions de l'ADN s'effectue à l'aide d'un logiciel qui permet de déterminer le pourcentage de la dégradation d'ADN.

Les résultats démontrent que les cellules soumises une privation de nutriments subissent un stress et que leur ADN est dégradé. En outre, les résultats démontrent que la protection de l'ADN des cellules traitées avec l'extrait de fibres de coton est augmentée de 45 % par rapport aux cellules contrôles (c'est-à-dire les cellules non traitées par l'extrait mais ayant subi le stress). L'extrait de fibres de coton joue donc un rôle important sur la protection de l'ADN.

### Exemple 6 - Test d'hydratation sur volontaires (test Clinique)

L'étude a été réalisée sur 8 volontaires, âgés de 21 à 39 ans, en double aveugle contre placebo.

L'extrait de fibres de coton selon l'exemple 1 a été administré par voie topique, à 1,5 % dans une formulation cosmétique standard pour obtenir une composition cosmétique. Une seule application a été réalisée par un expert.

Les évaluations ont été réalisées au temps 0 et 3 heures

La peau a été observée par une technique non invasive, à l'aide d'un Vivascope™.

Les critères quantitativement évalués ont été l'épaisseur du stratum corneum et l'épaisseur du stratum granulosum.

Résultat : Une diminution significative de l'épaisseur du stratum corneum a été observée après 3 heures d'application de la composition cosmétique comprenant l'extrait de fibres de coton.

| Epaisseur du Stratum corneum | Temps | % de diminution | % de volontaires améliorés |
|---|---|---|---|
| Composition avec extrait de fibres de coton | t3h-t0h | -250 % | 7/8 = 87,5 % |

Conclusion : La diminution de l'épaisseur du stratum corneum est une caractéristique de la bonne hydratation et de l'aspect sain de la peau. Les compositions selon l'invention permettent donc d'obtenir une bonne hydratation de la peau.

### Exemple 7 - Test d'hydratation sur volontaires (2eme protocole test Clinique)

L'étude a été réalisée sur 8 volontaires, âgés de 21 à 39 ans, en double aveugle contre placebo.

L'extrait de fibres de coton selon l'exemple 1 a été administré par voie topique, à 1,5 % dans une formulation cosmétique standard pour obtenir une composition cosmétique. Une seule application a été réalisée par un expert.

Les évaluations ont été réalisées aux temps 0 et 3 heures.

La méthode d'évaluation utilisée est le Cornéomètre CM825.

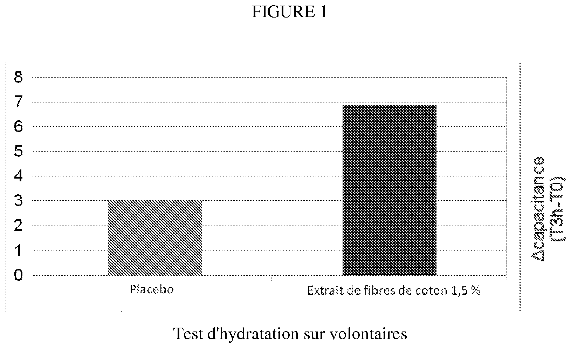

Les résultats sont présentés sur le graphe de la figure 1

### Résultats :

| Hydratation | Temps | Moyenne | +/- sem | p | % d'augmentation | % de volontaires améliorés |
|---|---|---|---|---|---|---|
| Placebo | t3h-t0h | 3.000 | 2.104 | | | |
| Composition avec extrait de fibres de coton | t3h-t0h | 6.875 | 2.117 | 0,0156* | 129.16 % | 6/8 = 75 % |

| | | | | | | |
|---|---|---|---|---|---|---|
| sem : erreur standard | | | | | | |

Conclusions : L'hydratation de la peau a été significativement augmentée après 3 heures d'application de l'extrait de fibres de coton. Les compositions selon l'invention permettent donc d'obtenir une bonne hydratation de la peau.

### Exemple 8 - Préparations de compositions

### 1 - Composition 1

| ***Ingrédients (nom de marque)*** | ***Ingrédients (nom INCI)*** | ***% w*/*w*** | ***Fournisseur*** |
|---|---|---|---|
| **Phase A** | | | |
| Eau purifiée | Eau | 64,57 | / |
| **Lubrasil™ II DM** | Glycerin (et) Glyceryl Acrylate/Acrylic Acid Copolymer (et) Laureth-23 (and) Dimethicone | 3 | **Ashland** |
| Na4EDTA | Tetrasodium EDTA | 0,15 | Fischer chemical |
| Lipoxol 3350 | PEG-75 | 1,5 | Sasol / IMCD |
| Glucam E-20 | Methylgluceth-20 | 3 | Unipex |
| **Lubrajel™ II XD Free** | Glycerin (and) Glyceryl Polyacrylate | 5 | **Ashland** |
| Solulan™C-24 Lanolin Derivative | Choleth-24 (et) Ceteth-24 | 1 | Unipex |
| **Lubrajel™ DV** | Glycerin (et) Glyceryl Acrylate/Acrylic Acid Copolymer (et) Propylene Glycol | 3 | **Ashland** |
| **Si-tec DMC 6038** | Bis-PEG-15 Methyl Ether Dimethicone | 3 | **Ashland** |

| **Phase B** | | | |
|---|---|---|---|
| **Ultrathix™ P100** | Acrylic Acid/VP Crosspolymer | 0,3 | **Ashland** |

| **Phase C** | | | |
|---|---|---|---|
| Cristalhyal | Sodium hyaluronate | 0,05 | Soliance |
| Eau purifiée | Eau | 10 | / |

| **Phase D** | | | |
|---|---|---|---|
| Solubilisant LRI | PPG-26 Buteth-26 (et) PEG-40 Hydrogenated Castor Oil (et) eau | 0,5 | Sensient |
| **Optiphen™** | Phenoxyethanol (et) Caprylyl glycol | 1,5 | **Ashland** |
| Belsil PDM 20 | Trimethylsiloxyphenyl Dimethicone | 1,5 | Wacker |
| PF Perfect smooth | Fragrance / Parfum | 0,2 | Mane |

| **Phase E** | | | |
|---|---|---|---|
| NaOH pearls | Sodium hydroxyde | 0,03 | Acros/ Fisc h er |
| Eau purifiée | Eau | 0,5 | / |

| **Phase F** | | | |
|---|---|---|---|
| Timiron Splendid Blue | CI77891 (Titanium Dioxyde) (et) Mica (et) Silica | 0,2 | Merck |
| EXTRAIT selon l'exemple 1 | | 0,1 | |
| **Total** | | **100,00** | |

### 2 - Emulsion huile dans eau

### Phase huileuse :

| | |
|---|---|
| ▪ Montanov 68 (Cetearyl Alcohol and Cetearyl Glucoside) | 5,00 % |
| ▪ Huile de Jojoba | 5,00 % |
| ▪ Huile de Vaseline | 5,00 % |
| ▪ Isopropyl Palmitate | 7,00 % |

### Phase aqueuse :

| | |
|---|---|
| ▪ Glycérine | 5,00 % |
| ▪ Allantoïne | 0,10 % |
| ▪ EXTRAIT selon l'exemple 1 | 10 % |
| ▪ Sepigel 305 (Polyacrylamide and C13-14 Isoparaffin and Laureth-7) | 0,30 % |
| ▪ Conservateur | 0,50 % |
| ▪ Parfum | 0,50 % |
| ▪ Eau | qsp 100 % |

### 3 - Gel

| | |
|---|---|
| ▪ Carbopol Ultrez 10 (sol. à 2%) | 25,00 % |
| ▪ Triéthanolamine | 0,50 % |
| ▪ EXTRAIT selon l'exemple 1 | 0.5 % |
| ▪ Conservateur | 0,20 % |
| ▪ EDTA (séquestrant) | 0,10 % |
| ▪ Parfum | 0,50 % |
| ▪ Eau | qsp 100 % |

### 4 - Lotion

| | |
|---|---|
| ▪ Mono Propylene Glycol | 1,00 % |
| ▪ Allantoïne | 0,30 % |
| ▪ Glycérine | 1,00 % |
| ▪ Cetiol HE (PEG-7 Glyceryl Cocoate) | 1,00 % |
| ▪ EXTRAIT selon l'exemple 1 | 0.01 % |
| ▪ Conservateur | 0,20 % |
| ▪ Parfum | 0,50 % |
| ▪ Eau | qsp 100 % |

### 5 - Shampooing

| | |
|---|---|
| ▪ Texapon NSO (Sodium Laureth Sulfate) | 30,00 % |
| ▪ Tegobetaïne HS (Cocamidopropyl Betaïne) | 6,00 % |
| ▪ Tween 20 | 2,00 % |
| ▪ Glucamate DOE 120 (sol à 50%) | 0,75 % |
| ▪ EDTA | 0,10 % |
| ▪ Chlorure de Sodium | 1,00 % |
| ▪ EXTRAIT selon l'exemple 1 | 0,5 - 1 % |
| ▪ Conservateur | 0,30 % |
| ▪ Parfum | 0,50 % |
| ▪ Colorant | qsp 100 % |
| ▪ Eau | qsp 100 % |
| ▪ Acide Citrique | qsp pH = 5,5 - 6,0 |

## Revendications

1. Composition cosmétique comprenant comme principe actif, dans un milieu physiologiquement adapté, entre 0,1 % et 10 % en poids par rapport au poids total de la composition d'un extrait de fibres de coton **caractérisé en ce que** ledit extrait de fibres de coton comprend 2 % en poids d'hydrolysat de fibres de coton constitué exclusivement de monosaccharides et/ou disaccharides et/ou trisaccharides et/ou tétrasaccharides, lesdits saccharides étant le glucose et/ou le cellobiose et/ou le cellotriose et/ou le cellotétraose et/ou des oligomères de cellulose, et que ledit extrait de fibres de coton comprend en outre 0,8 % en poids de glucose, 0,8 % en poids de fructose, 1,1 % en poids de tréhalose, 1,7 % en poids de saccharose et 0,5 % en poids d'inositol.

2. Composition selon la revendication 1, **caractérisée en ce qu'**elle est adaptée à une administration topique et se présente notamment sous forme d'une solution aqueuse, hydro-alcoolique ou huileuse ou sous la forme d'une émulsion huile-dans-eau, eau-dans-huile ou émulsions multiples ou sous forme de crèmes, de suspensions, ou encore de poudres ; ladite composition pouvant être plus ou moins fluide ou solide et avoir l'aspect d'une crème, d'une lotion, d'un lait, d'un shampooing, d'un sérum, d'une pommade, d'un gel, d'une pâte, d'une mousse ou d'un stick.

3. Composition pour utilisation dans une méthode pour protéger les substrats kératiniques, et plus particulièrement pour protéger la peau et les cheveux, contre tous les types d'agressions extérieures consistant à appliquer sur la surface des substrats kératiniques, et plus particulièrement sur la peau et/ou les cheveux, une quantité efficace d'une composition telle que définie dans l'une des revendications 1 ou 2.

4. Procédé de traitement cosmétique pour renforcer la barrière cutanée de la peau et/ou pour renforcer la protection des cheveux et/ou pour augmenter la synthèse de kératine et/ou pour nourrir les substrats kératiniques, et plus particulièrement la peau et les cheveux, consistant à appliquer sur la surface de la peau et/ou des cheveux, une quantité efficace d'une composition telle que définie dans l'une des revendications 1 ou 2.

## Patentansprüche

1. Kosmetische Zusammensetzung, umfassend als Wirkstoff in einem physiologisch akzeptablen Medium, zwischen 0,1 Gew.-% und 10 Gew.-%, bezogen auf das Gesamtgewicht der Zusammensetzung, eines Baumwollfaserextrakts, **dadurch gekennzeichnet, dass** der Baumwollfaserextrakt 2 Gew.-% Baumwollfaserhydrolysat umfasst, das ausschließlich aus Monosacchariden und/oder Disacchariden und/oder Trisacchariden und/oder Tetrasacchariden besteht, wobei die Saccharide Glucose und/oder Cellobiose und/oder Cellotriose und/oder Cellotetraose und/oder Celluloseoligomere sind, und dadurch, dass der Baumwollfaserextrakt ferner 0,8 Gew.-% Glucose, 0,8 Gew.-% Fructose, 1,1 Gew.-% Trehalose, 1,7 Gew.-% Saccharose und 0,5 Gew.-% Inositol umfasst.

2. Zusammensetzung nach Anspruch 1, **dadurch gekennzeichnet, dass** sie zur topischen Verabreichung geeignet ist und insbesondere in Form einer wässrigen, hydroalkoholischen oder ölbasierten Lösung oder in Form einer Öl-in-Wasser-, Wasserin-Öl-Emulsion oder von Mehrfachemulsionen oder in Form von Cremen, Suspensionen oder auch Pulvern vorliegt; wobei die Zusammensetzung mehr oder weniger flüssig oder fest sein kann und das Aussehen einer Creme, einer Lotion, einer Milch, eines Shampoos, eines Serums, einer Salbe, eines Gels, einer Paste, eines Schaums oder eines Stifts aufweisen kann.

3. Zusammensetzung zur Verwendung in einem Verfahren zum Schutz von Keratinsubstraten und insbesondere zum Schutz von Haut und Haaren gegen alle Arten von äußeren Einflüssen, das darin besteht, auf die Oberfläche der Keratinsubstrate und insbesondere auf die Haut und/oder die Haare eine wirksame Menge einer Zusammensetzung nach einem der Ansprüche 1 oder 2 aufzubringen.

4. Kosmetisches Behandlungsverfahren zur Stärkung der kutanten Barriere der Haut und/oder zur Stärkung des Haarschutzes und/oder zur Erhöhung der Keratinsynthese und/oder zur Ernährung der Keratinsubstrate und insbesondere der Haut und der Haare, das darin besteht, auf die Oberfläche der Haut und/oder der Haare eine wirksame Menge einer Zusammensetzung nach einem der Ansprüche 1 oder 2 aufzutragen.

## Claims

1. A cosmetic composition comprising, as active ingredient, in a physiologically acceptable medium, between 0.1 % and 10 % by weight, in relation to the total weight of the composition, of a cotton fibres extract, **characterised in that** said cotton fibres extract comprises 2 % by weight of a cotton fibres hydrolysate formed exclusively of monosaccharides and/or disaccharides and/or trisaccharides and/or tetrasaccharides, said saccharides being glucose and/or cellobiose and/or cellotriose and/or cellotetraose and/or cellulose oligomers, and **in that** said cotton fibres extract also comprises 0.8 % by weight of glucose, 0.8 % by weight of fructose, 1.1 % by weight of trehalose, 1.7 % by weight of sucrose, and 0.5 % by weight of inositol.

2. The composition according to claim 1, **characterised in that** it is suitable for topical administration and is present in particular in the form of an aqueous, hydro-alcoholic or oil-based solution, or in the form of an oil-in-water emulsion, water-in-oil emulsion, or multiple emulsions, or in the form of creams, suspensions, or also powders, said composition possibly being fluid or solid to a greater or lesser extent and having the appearance of a cream, a lotion, a milk, a shampoo, a serum, an ointment, a gel, a paste, a foam or a stick.

3. A composition for use in a method for protecting keratin substrates, and more particularly for protecting skin and hair, against any types of external attacks, consisting of applying to the surface of the keratin substrates, and more particularly to skin and/or hair, an effective quantity of a composition as defined in either one of claims 1 or 2.

4. A cosmetic treatment method for reinforcing the skin barrier and/or for reinforcing hair protection and/or for increasing keratin synthesis and/or for nourishing keratin substrates, and more particularly the skin and hair, consisting of applying to the surface of the skin and/or hair, an effective quantity of a cosmetic composition as defined in either one of claims 1 or 2.
